# EUROPÄISCHE PATENTANMELDUNG

(11) **EP 0 839 552 A2**
(43) Veröffentlichungstag der Anmeldung: **06.05.1998**
(21) Anmeldenummer: 97118118.5
(22) Anmeldetag: 18.10.1997
(51) Int. Cl.: A61N 1/05

(54) **Elektrode für die Implantation in Körpergewebe, Verfahren zu ihrer Herstellung und ihre Verwendung (II)**

(30) Priorität: 02.11.1996 DE 19645162
(71) Anmelder: W.C. Heraeus GmbH, D-63450 Hanau (DE)
(72) Erfinder: Herklotz, Günther, Dr., 63486 Bruchköbel (DE); Schölz, Friedhold, 63517 Rodenbach (DE); Giesel, Thomas, 63526 Erlensee (DE)
(74) Vertreter: Kühn, Hans-Christian

(57) **Zusammenfassung**

An einer Elektrode, deren aktiver Bereich eine aus mehreren Schichten bestehende poröse, strukturierte Beschichtung mit großer Oberfläche aufweist, bildet sich eine hohe Doppelschichtkapazität aus. Die Elektrode ist besonders zur Verwendung in Herzschrittmachern geeignet.

## Beschreibung

Die Erfindung betrifft eine Elektrode für die Implantation in Körpergewebe aus einem metallischen Grundkörper und einer den aktiven Bereich der Elektrode bildenden porösen Beschichtung, ein Verfahren zu ihrer Herstellung und ihre Verwendung.

Die Erfindung betrifft besonders eine Elektrode für Herzschrittmacher.

Implantierbare Elektroden dieser Art bestehen in den meisten Fällen aus einem Elektrodenschaft mit einer isolierten Kabelzuleitung und aus einem Elektrodenkopf - dem aktiven oder wirksamen Bereich der Elektrode. Das Elektrodenmaterial muß körperverträglich sein, um die Bildung von Bindegewebsschichten soweit wie möglich zu verhindern und die Reizschwelle möglichst konstant zu halten. An der Phasengrenze Elektrode/Körperflüssigkeit soll sich eine hohe Doppelschichtkapazität ausbilden; der Polarisationsanstieg während der Reiz- oder Stimulationsimpulse soll kleiner als 0,1 V sein. Die geforderte hohe Doppelschichtkapazität wirkt sich bei Reizelektroden (Stimulationselektroden) günstig aus, weil durch den aufgeprägten Strom nur geringe Potentialänderungen entstehen, elektrochemische Reaktionen mit der Körperflüssigkeit weitgehend unterbleiben und der Energieaufwand gering ist. Im Falle von Sensoren, bei denen nur ein kleiner Meßstrom fließt, erleichtert eine hohe Kapazität der Elektroden die Erfüllung der Anforderungen, die an die Eingangsimpedanz von Verstärkern zu stellen sind; außerdem wird das Rauschen reduziert (DE 33 00 668 A1).

Implantierbare Elektroden mit porösen Oberflächen sind zum Beispiel aus GB 1 552 388, EP 0 043 461 A1, EP 0 054 781 B1, DE 33 00 668 A1 und DE 42 07 368 A1 bekannt.

In GB 1 552 388 werden kardiovaskuläre Prothesen und Implantate, beispielsweise Teile für Herzschrittmacherelektroden, mit porösen metallischen Überzügen beschrieben. Die Überzüge bestehen aus miteinander verbundenen metallischen Teilchen, die ein sich gleichmäßig verteilendes Porennetz bilden. Die Überzüge werden durch Aufbringen der metallischen Teilchen auf die aufgerauhte Oberfläche der metallischen Grundkörper und anschließendes Sintern hergestellt. Die Art und Weise des Aufbringens der metallischen Teilchen hängt von deren Korngröße ab und kann zum Beispiel durch Aufsprühen einer viskosen Mischung aus metallischen Teilchen und Bindemittel und nachfolgendes Trocknen erfolgen. Die für das Sintern günstige Temperatur hängt ebenfalls von der Korngröße der metallischen Teilchen ab und liegt zwischen 1100 und 1200° C.

Aus EP 0 043 461 A1 ist eine Elektrode für Herzstimulatoren bekannt, deren Kopf aus einer mit einem Überzug aus Platin-Schwamm versehenen Platin-Iridium-Legierung besteht. Die Dicke des Überzugs beträgt etwa 0,1 Millimeter, der Porendurchmesser etwa 40 Mikrometer. Der Überzug aus Platin-Schwamm wird durch galvanisches Abscheiden erzeugt und kann durch Sintern bei etwa 1300° C verdichtet werden.

In EP 0 054 781 B1 wird eine poröse Elektrode zur Implantation in organisches Gewebe vorgeschlagen, die als Grundkörper einen aus elektrisch leitenden Teilchen hergestellten Sinterkörper aufweist. Eine vorteilhafte Ausführungsform der Elektrode besteht aus einem Tantal-Sinterkörper, dessen Oberfläche teilweise mit einer dünnen Tantaloxid-Schicht und teilweise mit einer aus einem handelsüblichen Platin-Bad galvanisch abgeschiedenen Platin-Schicht versehen ist. Der größte Teil der Poren ist zum Körpergewebe hin offen; die Porengröße hat vorteilhafterweise einen Durchmesser im Bereich von 10 - 50 Mikrometer. Die Elektrode besitzt eine niedrige Reizschwelle und stellt besonders eine Herzschrittmacherelektrode dar.

In DE 33 00 668 A1 wird eine Elektrode für medizinische Anwendungen, besonders eine implantierbare Reizelektrode, beispielsweise für Herzschrittmacher, beschrieben. Sie besteht aus einem elektrisch leitenden Trägermaterial aus zum Beispiel Titan oder Platin und weist im aktiven Bereich eine poröse Schicht aus einem Carbid, Nitrid oder Carbonitrid eines oder mehrerer der Übergangsmetalle Titan, Vanadin, Zirkonium, Niob, Molybdän, Hafnium, Tantal und Wolfram mit einer Dicke zwischen 1 und 100 Mikrometer auf. Die poröse Schicht wird vorzugsweise durch reaktives Ionenplattieren (physikalische Dampfabscheidung) aufgebracht. Zwischen dem Trägermaterial und der porösen Schicht kann sich zusätzlich eine dichte Schicht, vorzugsweise aus dem die poröse Schicht bildenden Material, befinden, so daß das Trägermaterial nicht unbedingt körperverträglich sein muß. Die potentiostatisch aus Impedanzmessungen ermittelte Doppelschichtkapazität einer Elektrode aus mit porösem Titannitrid beschichtetem Titan beträgt 21,5 mF/cm² bei einer Frequenz von 1 Hz.

Aus DE 42 07 368 A1 ist eine zur Anwendung als Herzschrittmacher- oder Neurostimulationselektrode geeignete Stimulationselektrode aus einem Grundkörper und einer porösen Oberflächenbeschichtung aus inertem Material mit nur sehr geringer Oxidationsneigung bekannt. Das inerte Material kann ein inertes Element, eine inerte chemische Verbindung und/oder eine inerte Legierung sein und besteht besonders aus einem Carbid, Nitrid oder Carbonitrid, aus Gold, Silber, Iridium, Platin, einer Legierung dieser Metalle oder aus Kohlenstoff und wird durch Vakuumbeschichtungsverfahren (reaktive Kathodenzerstäubung oder Ionenplattierung) auf den Grundkörper aufgebracht. Wie die elektronenmikroskopisch vergrößerte Darstellung zeigt, besitzt die Stimulationselektrode ein blumenkohlartiges Äußeres. Durch die sich nach außen hin verfeinernde Struktur wird eine mikroskopische Oberfläche erzielt, die um ein Vielfaches größer ist als der makroskopische Flächenbereich. Da die aktive Oberfläche der Elektrode sehr groß ist, kann die zur Stimulation erforderliche Energiemenge gering gehalten werden, so daß die Betriebszeit (Lebensdauer) der die Elektrode enthaltenden Implantate verlängert wird. Mit den Elektroden - sie können monopolar, bipolar oder multipolar sein - kann auch eine infrakardiale Impedanzmessung zur Erfassung der Herzaktivität erfolgen. Eine mit Iridiumnitrid beschichtete Elektrode besitzt in dem für den Empfang von aus dem Herzen aufnehmenden Signalen besonders wichtigen niederfrequenten Bereich eine sehr niedrige Phasengrenzimpedanz.

Der Erfindung liegt die Aufgabe zugrunde, eine Elektrode für die Implantation in Körpergewebe zur Verfügung zu stellen, die aus einem metallischen Grundkörper und einer den aktiven Bereich der Elektrode bildenden porösen Beschichtung besteht. Die poröse Beschichtung soll so strukturiert sein, daß eine große aktive Oberfläche und eine an der Elektrode sich ausbildende hohe Doppelschichtkapazität erreicht werden. Außerdem soll ein Verfahren zur Herstellung der Elektrode, die besonders zur Verwendung in Herzschrittmachern geeignet sein soll, angegeben werden.

Die die Lösung der Aufgabe darstellende Elektrode ist erfindungsgemäß dadurch gekennzeichnet, daß die Beschichtung eine direkt auf dem Grundkörper befindliche poröse, stengelartig strukturierte Platin-Schicht und darauf eine poröse, feinststrukturierte Schicht aus einem Carbid, Nitrid und/oder Carbonitrid eines Übergangsmetalls aufweist.

Besonders bewährt hat sich die erfindungsgemäße Elektrode, wenn die Dicke der porösen Beschichtung 10 - 200 Mikrometer, vorzugsweise 50 - 100 Mikrometer, beträgt und die Dicke der porösen, stengelartig strukturierten Platin-Schicht 80 - 90 % der Dicke der gesamten Beschichtung ausmacht.

Die Charakterisierung der die Beschichtung bildenden Schichten im Sinne der Erfindung als poröse, stengelartig strukturierte Platin-Schicht und als poröse, feinststrukturierte Carbid-, Nitrid- und/oder Carbonitrid-Schicht beruht auf der mikroskopischen Betrachtung der Beschichtung.

Der metallische Grundkörper besteht aus einem körperverträglichen Übergangsmetall oder einer körperverträglichen Übergangsmetall-Legierung. Als besonders geeignet haben sich Titan und Platin-Iridium-Legierungen mit bis zu 20 Gewichts-% Iridium, zum Beispiel eine Legierung aus 90 Gewichts-% Platin und 10 Gewichts-% Iridium, erwiesen.

Für die poröse, feinststrukturierte Schicht eignen sich die Carbide, Nitride und/oder Carbonitride von Übergangsmetallen der Gruppen IVA bis VIA des Periodensystems. Besonders vorteilhaft läßt sich die Elektrode einsetzen, wenn das Übergangsmetall aus Titan, Tantal oder Titan und Tantal besteht. Bevorzugt werden - einzeln oder im Gemisch miteinander - Titannitrid, Titancarbonitrid, Tantalnitrid und Tantalcarbonitrid.

Weiterhin wird die Aufgabe der Erfindung durch ein Verfahren gelöst, nach dem die beanspruchte Elektrode hergestellt werden kann.

Dieses Verfahren ist erfindungsgemäß dadurch gekennzeichnet, daß auf das den aktiven Elektroden-Bereich bildende Teil des Grundkörpers eine Platin-Pulver enthaltende pastenförmige Zubereitung und darauf Platin-Pulver aufgebracht, der so behandelte Grundkörper getrocknet und gesintert und anschließend eine poröse, feinststrukturierte Schicht aus einem Carbid, Nitrid- und/oder Carbonitrid eines Übergangsmetalls vakuumtechnisch aufgebracht wird.

Das erfindungsgemäße Verfahren wird vorzugsweise so ausgeführt, daß die Dicke der fertigen Beschichtung 10 - 200 Mikrometer, besonders 50 - 100 Mikrometer, beträgt. Dabei erfolgt das Auftragen von pastenförmiger Zubereitung und Platin-Pulver so, daß die Dicke der daraus durch Trocknen und Sintern erzeugten porösen, stengelartig strukturierten Platin-Schicht 80 bis 90 % der Dicke der Beschichtung ausmacht.

Das erfindungsgemäße Verfahren hat sich besonders bewährt, wenn das Aufbringen von pastenförmiger Zubereitung und Platin-Pulver auf den metallischen Grundkörper und das Trocknen und Sintern mindestens einmal wiederholt werden und das Trocknen bei 100 - 200° C und das Sintern bei 400 - 1300° C erfolgt.

Für das Verfahren wird eine pastenförmige Zubereitung, die neben einem thermisch zersetzbaren organischen Bindemittel, besonders aus einem Cellulosederivat, und einem organischen Träger 20 - 80 Gewichts-% Platin-Pulver enthält, bevorzugt. Sowohl für die pastenförmige Zubereitung als auch zum Auftragen darauf hat sich ein Platin-Pulver, dessen Teilchengröße 0,001 - 10 Mikrometer beträgt, als besonders geeignet erwiesen.

Der in dem Verfahren eingesetzte metallische Grundkörper besteht aus einem körperverträglichen Übergangsmetall oder einer körperverträglichen Übergangsmetall-Legierung, vorzugsweise aus Titan oder einer Platin-Iridium-Legierung mit bis zu 20 Gewichts-% Iridium, zum Beispiel einer Legierung aus 90 Gewichts-% Platin und 10 Gewichts-% Iridium.

Die poröse, feinststrukturierte Schicht aus dem Übergangsmetallcarbid, -nitrid und/oder -carbonitrid wird vorzugsweise in an sich bekannter Weise durch reaktive Kathodenzerstäubung aufgebracht. Besonders bewährt hat sich das Aufbringen von Titannitrid, Titancarbonitrid, Tantalnitrid und/oder Tantalcarbonitrid.

Gegebenenfalls kann zusätzlich noch eine poröse Schicht aus feindispersem Platin, wie in EP 0 043 461 A1 beschrieben, durch galvanisches Abscheiden aufgebracht und durch Sintern verdichtet werden.

Die Elektrode gemäß der Erfindung zeichnet sich durch die an der Grenzfläche Elektrode/Elektrolyt sich ausbildende hohe Doppelschichtkapazität aus, die im wesentlichen auf der porösen Beschichtung mit der ihr eigenen Strukturierung beruht.

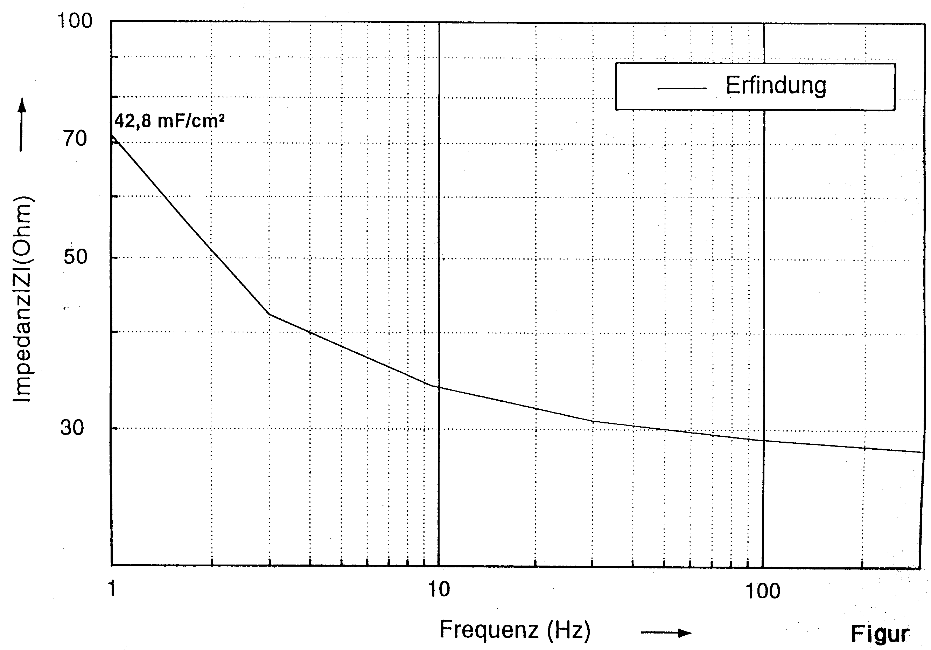

Für die Bestimmung der üblicherweise zur Charakterisierung implantierbarer Elektroden dienenden Doppelschichtkapazität bei 1 Hz wird als beispielhafte Ausführungsform der Erfindung eine Elektrode eingesetzt, deren Grundkörper aus Titan und deren aktiver Bereich aus einer porösen Beschichtung aus einer 50 Mikrometer dicken porösen, stengelartig strukturierten Platin-Schicht und einer 10 Mikrometer dicken porösen, feinststrukturierten Titannitrid-Schicht besteht. Die zur Bestimmung der Doppelschichtkapazität angewandten Messungen der Impedanz [Ohm] in Abhängigkeit von der Frequenz [Hz] erfolgen in 0,15 n NaCl-Lösungen. In der Figur werden die in Abhängigkeit von der Frequenz gemessenen Impedanz-Werte dargestellt.

Die anhand der Impedanzmessungen für die erfindungsgemäße Elektrode (A) bestimmte Doppelschichtkapazität und - zum Vergleich dazu - die der aus DE 33 00 668 A1 bekannten Elektrode (B) mit aus Titannitrid bestehender Beschichtung werden in der Tabelle angegeben.

| Tabelle | |
|---|---|
| **Elektrode** | **Doppelschichtkapazität bei 1 Hz [mF/cm**^{**2**}**]** |
| A Erfindung | 42,8 |
| B Vergleich | 21,5 |

## Patentansprüche

1. Elektrode für die Implantation in Körpergewebe aus einem metallischen Grundkörper und einer den aktiven Bereich der Elektrode bildenden porösen Beschichtung, dadurch gekennzeichnet, daß die Beschichtung eine direkt auf dem Grundkörper befindliche poröse, stengelartig strukturierte Platin-Schicht und darauf eine poröse, feinststrukturierte Schicht aus einem Carbid, Nitrid und/oder Carbonitrid eines Übergangsmetalls aufweist.

2. Elektrode nach Anspruch 1, dadurch gekennzeichnet, daß die poröse, stengelartig strukturierte Platin-Schicht gesintert ist.

3. Elektrode nach Anspruch 1 oder 2, dadurch gekennzeichnet, daß die Dicke der Beschichtung 10 - 200 Mikrometer beträgt.

4. Elektrode nach Anspruch 3, dadurch gekennzeichnet, daß die Dicke der Beschichtung 50 - 100 Mikrometer beträgt.

5. Elektrode nach einem der Ansprüche 1 bis 4, dadurch gekennzeichnet, daß die Dicke der porösen, stengelartig strukturierten Platin-Schicht 80 - 90 % der Dicke der Beschichtung ausmacht.

6. Elektrode nach einem der Ansprüche 1 bis 5, dadurch gekennzeichnet, daß der metallische Grundkörper aus einem körperverträglichen Übergangsmetall besteht.

7. Elektrode nach Anspruch 6, dadurch gekennzeichnet, daß der metallische Grundkörper aus Titan besteht.

8. Elektrode nach Anspruch 6, dadurch gekennzeichnet, daß der metallische Grundkörper aus einer Platin-Iridium-Legierung besteht.

9. Elektrode nach einem der Ansprüche 1 bis 8, dadurch gekennzeichnet, daß die poröse, feinststrukturierte Schicht aus Titannitrid, Titancarbonitrid, Tantalnitrid und/oder Tantalcarbonitrid besteht.

10. Elektrode nach einem der Ansprüche 1 bis 9, dadurch gekennzeichnet, daß sich auf der porösen, feinststrukturierten Schicht eine poröse Schicht aus feindispersem Platin befindet.

11. Verfahren zur Herstellung der Elektrode nach einem der Ansprüche 1 bis 10, dadurch gekennzeichnet, daß auf das den aktiven Elektroden-Bereich bildende Teil des metallischen Grundkörpers eine Platin-Pulver enthaltende pastenförmige Zubereitung und darauf Platin-Pulver aufgebracht, der so behandelte Grundkörper getrocknet und gesintert und anschließend eine poröse, feinststrukturierte Schicht aus einem Carbid, Nitrid und/oder Carbonitrid eines Übergangsmetalls vakuumtechnisch aufgebracht wird.

12. Verfahren nach Anspruch 11, dadurch gekennzeichnet, daß das Aufbringen von pastenförmiger Zubereitung und Platin-Pulver und das Trocknen und Sintern mindestens einmal wiederholt werden.

13. Verfahren nach Anspruch 11 oder 12, dadurch gekennzeichnet, daß das Trocknen bei 100 - 200° C und das Sintern bei 400 - 1300° C erfolgt.

14. Verfahren nach einem der Ansprüche 11 bis 13, dadurch gekennzeichnet, daß die pastenförmige Zubereitung 20 - 80 Gewichts-% Platin-Pulver enthält.

15. Verfahren nach einem der Ansprüche 11 bis 14, dadurch gekennzeichnet, daß die Teilchengröße des Platin-Pulvers 0,001 - 10 Mikrometer beträgt.

16. Verfahren nach einem der Ansprüche 11 bis 15, dadurch gekennzeichnet, daß die pastenförmige Zubereitung ein organisches Bindemittel enthält.

17. Verfahren nach Anspruch 16, dadurch gekennzeichnet, daß das organische Bindemittel aus einem Cellulosederivat besteht.

18. Verfahren nach einem der Ansprüche 11 bis 17, dadurch gekennzeichnet, daß eine poröse, feinststrukturierte Schicht aus Titannitrid, Titancarbonitrid, Tantalnitrid und/oder Tantalcarbonitrid aufgebracht wird.

19. Verfahren nach einem der Ansprüche 11 bis 18, dadurch gekennzeichnet, daß auf der porösen, feinststrukturierten Schicht eine poröse Schicht aus feindispersem Platin galvanisch abgeschieden wird.

20. Verfahren nach Anspruch 19, dadurch gekennzeichnet, daß die poröse Schicht aus feindispersem Platin gesintert wird.

21. Verfahren nach einem der Ansprüche 11 bis 20, dadurch gekennzeichnet, daß ein Grundkörper aus körperverträglichem Übergangsmetall eingesetzt wird.

22. Verfahren nach Anspruch 21, dadurch gekennzeichnet, daß ein Grundkörper aus Titan eingesetzt wird.

23. Verfahren nach Anspruch 21, dadurch gekennzeichnet, daß ein Grundkörper aus einer Platin-Iridium-Legierung eingesetzt wird.

24. Verwendung der Elektrode nach einem der Ansprüche 1 bis 10 in Herzschrittmachern.

25. Verwendung der Elektrode nach einem der Ansprüche 1 bis 10 als Stimulationselektrode.
